# EUROPEAN PATENT APPLICATION

(11) **EP 3 202 329 A1**
(43) Date of publication of application: **09.08.2017**
(21) Application number: 15846455.2
(22) Date of filing: 25.09.2015
(51) Int. Cl.: A61B 8/14

(54) **ULTRASONIC IMAGE PROCESSING DEVICE**

(30) Priority: 01.10.2014 JP 2014203101
(71) Applicant: Hitachi, Ltd., Chiyoda-ku, Tokyo 100-8280 (JP)
(72) Inventor: KOBAYASHI Masaki, Mitaka-shi Tokyo 181-8622 (JP); INOUE Nobuyasu, Mitaka-shi Tokyo 181-8622 (JP); MURASHITA Masaru, Mitaka-shi Tokyo 181-8622 (JP); NAGASE Yuko, Mitaka-shi Tokyo 181-8622 (JP)
(74) Representative: Strehl Schübel-Hopf & Partner
(86) International application number: PCT/JP2015/077179
(87) International publication number: WO 2016/052354

(57) **Abstract**

According to the present invention, when measurement of a distance (fetal head biparietal diameter) on an ultrasonic image is started, a first mobile marker for designating a start point and a standard range display graphic are displayed. The standard range display graphic is a graphic that is centered on the first mobile marker and comprises two circular graphics having radii that are configured to be a lower limit and upper limit of a fetal head biparietal diameter standard range. A user can designate the start point while recognizing, via the standard range display graphic, an end point candidate range. Subsequently, an end point can be designated while recognizing the end point candidate range via a stationary standard range display graphic.

## Description

### TECHNICAL FIELD

The present disclosure relates to an ultrasound image processor, and in particular to an ultrasound image processor which executes a distance measurement on an ultrasound image.

### BACKGROUND

An ultrasound image is formed by an ultrasound diagnostic apparatus which transmits and receives ultrasound to and from a subject. The ultrasound image is used for various examinations and measurements. For example, in order to check a growth state of a fetus, a diameter of a head of the fetus (fetal biparietal diameter) is measured. In this measurement, on an ultrasound image (B-mode image) including a cross section of the head of the fetus, a distance from a cranial bone at a right side head to a cranial bone at a left side head is measured.

More specifically, in general, on a B-mode image displayed on a display, two measurement points are sequentially designated by a user such as a doctor, and a distance between the two measurement points is automatically calculated.

There may be cases where a large number of artifacts exist in the ultrasound image. In such cases, a tissue outline becomes unclear on the ultrasound image, and it becomes difficult for the user to accurately designate individual measurement points. If correct designation of both measurement points becomes impossible, a problem of erroneous measurement may result.

Patent Document 1 discloses a technique for supporting the positioning of the measurement point on the ultrasound image. In an apparatus disclosed in Patent Document 1, in the measurement of the fetal biparietal diameter, after a starting point is designated, a guide showing a normal range of growth of the fetus is displayed on a screen. The guide includes a line showing a direction passing from the starting point through an end point determining marker, and a line pair including two lines which are orthogonal to the line, which are relatively short, and which show an upper limit and a lower limit of a normal range. The user can use the guide as an estimate, to designate the position of the end point. With such a configuration, erroneous designation of the end point position is prevented.

### CITATION LIST

### PATENT LITERATURE

Patent Document 1: JP 4149015 B

### SUMMARY

### TECHNICAL PROBLEM

In the technique of Patent Document 1, because the guide is displayed after the starting point is designated, a support for positioning the starting point cannot be obtained. Therefore, in cases where a large number of artifacts exist in the ultrasound image, it is difficult to accurately position the starting point. When the user fails in the positioning of the starting point; that is, when the user designates, as the starting point, a position which is not a position to be set as the measurement point, the user may then recognize the possibility that the user failed to position the starting point by viewing a relationship between the displayed guide and an outline position which is a candidate of the end point, but such a recognition can only be obtained after the starting point is designated. In this case, the user must cancel the starting point position which is already designated, and re-designate the starting point position, which results in an unnecessary effort for the user.

An advantage of the present disclosure lies in supporting designation of two measurement points (in particular, the starting point) in an ultrasound image processor which measures a distance between the two measurement points (starting point and end point) designated on an ultrasound image. Another advantage of the present disclosure lies in allowing designation of the starting point while recognizing a range that can be taken by the end point on a tissue structure appearing as an ultrasound image.

### SOLUTION TO PROBLEM

According to one aspect of the present disclosure, there is provided an ultrasound image processor comprising: a measurement unit that measures a distance between a first measurement point and a second measurement point which are sequentially designated on an ultrasound image; a first marker displaying unit that displays a first marker which moves on the ultrasound image in response to a user operation for designating the first measurement point; and an index figure displaying unit that displays, at least before the first measurement point is designated and on the ultrasound image, an index figure showing a standard range of a distance between the first measurement point and the second measurement point with the first marker serving as a point of reference.

According to the above-described configuration, in the process of designating the first measurement point (starting point), the index figure is displayed on the ultrasound image along with the first marker. Through the index figure, the user can designate the first measurement point while recognizing a candidate range for the second measurement point; that is, a candidate range for the end point. In other words, the user can comprehensively evaluate validity of the position of the first measurement point to be currently designated while recognizing the standard range which can be taken by the second measurement point in relation to the tissue structure displayed as the ultrasound image; that is, while considering the validity of the position of the second measurement point to be designated in the future. Therefore, according to the above-described configuration, the user's load upon designating the first measurement point can be reduced, and a designation precision of the first measurement point can be improved.

When the display of the index figure is maintained even after the designation of the first measurement point, the support by the index figure can be continued for the designation of the second measurement point. The index figure may be expressed in various forms so long as the index figure can show the standard range for the distance to be measured. Desirably, the index figure includes two figures showing an upper limit and a lower limit of the standard range, and the concept includes a band-shaped figure having two edges.

According to another aspect of the present disclosure, the index figure includes a first two-dimensional shape having a circular shape or an arc shape centered at the first marker and having a lower limit value of the standard range as a radius, and a second two-dimensional shape having a circular shape or an arc shape centered at the first marker and having an upper limit value of the standard range as a radius. In a stage before the starting point of the distance measurement is designated, the ultrasound image processor cannot know in what direction from the current position of the first marker the distance will be measured. Therefore, the index figure desirably has first and second two-dimensional shapes which are circular shapes or arc shapes, in order to show the standard distance range in a plurality of directions (desirably, all directions) from the first marker. Alternatively, the direction of distance measurement may be deduced based on the current position of the first marker or the like, and the index figure may be displayed only in the deduced direction.

According to another aspect of the present disclosure, the index figure displaying unit moves the index figure in a manner to follow a movement of the first marker.

According to another aspect of the present disclosure, the ultrasound image processor further comprises a second marker displaying unit that displays a second marker which moves on the ultrasound image in response to a user operation for designating the second measurement point after the first measurement point is designated, wherein the index figure displaying unit fixes display positions of the first two-dimensional shape and the second two-dimensional shape when the first measurement point is designated, and limits, when the second marker is moved after the display positions are fixed, display ranges of the first two-dimensional shape and the second two-dimensional shape to an angle range centered around a direction from the first measurement point and through a display position of the second marker.

When the starting point is designated, the display positions of the first and second two-dimensional shapes are fixed, and, even after the starting point is designated, the first and second two-dimensional shapes continue to be displayed. With such a configuration, the user can use the first and second two-dimensional shapes as an estimate for positioning the second measurement point (end point of distance measurement). After the starting point is designated, the second marker is displayed for designating the end point. The ultrasound image processor can deduce the direction of the distance measurement by the positional relationship between the starting point and the second marker. The index figure displaying unit thus limits the display ranges of the first and second two-dimensional shapes to an angle range centered around the direction deduced as the distance measurement direction. With such a configuration, while the first and second two-dimensional shapes are displayed as the estimate for positioning the end point, portions of the first and second two-dimensional shapes that are considered to be unnecessary are not displayed, to thereby resolve the complexity of display.

According to another aspect of the present disclosure, the index figure displaying unit reduces the angle range with increasing distance between the first measurement point and the second marker. Desirably, a lower limit value is set for the angle range, so that, even when the distance between the starting point position and the second marker is increased, the two-dimensional shapes remain to be displayed to a degree to allow use of the shapes as the estimate of the positioning of the end point.

According to another aspect of the present disclosure, the second marker displaying unit initially displays the second marker at the designated first measurement point, and moves, when the second marker is moved, the second marker in a direction of movement of the second marker from the first measurement point and to a range between the first two-dimensional shape and the second two-dimensional shape.

### ADVANTAGEOUS EFFECTS OF INVENTION

According to various aspects of the present disclosure, in an ultrasound image processor which executes distance measurement between two measurement points (starting point and end point) designated on an ultrasound image, designation of the two measurement points (in particular, the starting point) can be supported. Further, according to various aspects of the present disclosure, it becomes possible to designate a starting point while recognizing a range which can be taken by the end point on a tissue structure appearing as an ultrasound image.

### BRIEF DESCRIPTION OF DRAWINGS

FIG. 1 is a schematic diagram of a structure of an ultrasound diagnostic apparatus according to an embodiment of the present disclosure.
FIG. 2 is a diagram showing a function representing a relationship between a normal range of a fetal biparietal diameter and the number of weeks of pregnancy.
FIG. 3 is a diagram showing an example display of a standard range displaying figure showing a standard range of a fetal biparietal diameter.
FIG. 4 shows an example display when a first movable marker is positioned at a starting point candidate position.
FIG. 5A is a diagram showing another example usage of a circular figure.
FIG. 5B is a diagram showing another example usage of the circular figure.
FIG. 6A is a diagram showing another example display form of the circular figure.
FIG. 6B is a diagram showing another example display form of the circular figure.
FIG. 7 is a diagram showing an example display when a second movable marker is moved after a starting point is designated.
FIG. 8 is a diagram showing an example display when the second movable marker is further moved.
FIG. 9 is a diagram showing an alternative example of a movement method of the second movable marker.
FIG. 10 is a diagram showing an example display when a starting point is corrected after an end point is designated.
FIG. 11 is a flowchart showing another example flow of operations of an ultrasound diagnostic apparatus according to an embodiment of the present disclosure.

### DESCRIPTION OF EMBODIMENTS

An embodiment of the present disclosure will now be described with reference to the drawings.

An ultrasound diagnostic apparatus 10 is a medical device which is generally equipped in a medical institution such as a hospital, and which executes an ultrasound diagnosis for a subject. The ultrasound diagnostic apparatus 10 forms an ultrasound image used for various examinations and measurements. The ultrasound diagnostic apparatus 10 has a function to measure a distance between two measurement points designated on the ultrasound image which is formed. In the following, the distance measurement function of the ultrasound diagnostic apparatus 10 will be described exemplifying measurement of a fetal biparietal diameter for checking a growth state of a fetus. In the present specification, the ultrasound diagnostic apparatus 10 is one type of an ultrasound image processor.

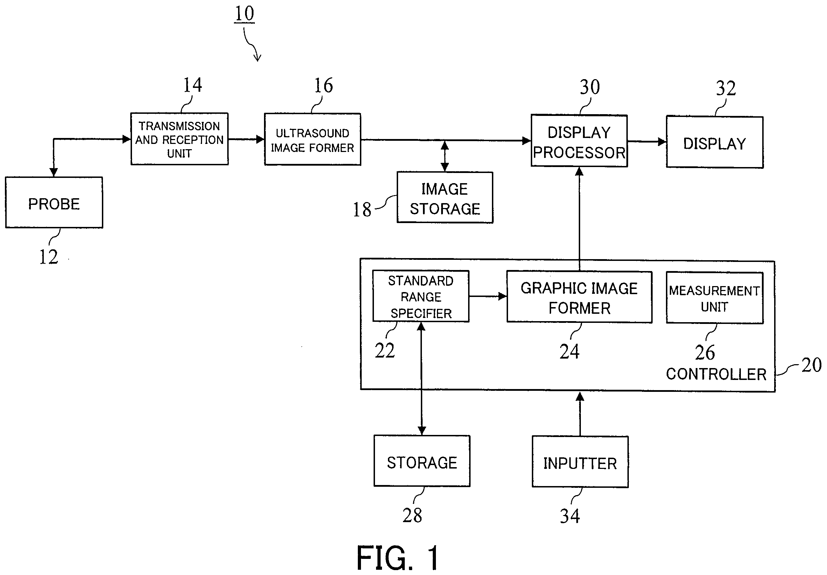

FIG. 1 is a schematic diagram of a structure of the ultrasound diagnostic apparatus 10. A probe 12 is an ultrasound probe which transmits and receives ultrasound to and from a subject. The probe 12 has a transducer array including a plurality of transducers. During transmission, a plurality of transmission signals are supplied from a transmission and reception unit 14 to the plurality of transducers of the transducer array, and the transducers are excited. With this process, an ultrasound beam (transmission beam) is produced. Meanwhile, during reception, when the plurality of transducers of the transducer array receive a reflection echo, in each transducer an acoustic signal is converted into a reception signal, which is an electric signal. A plurality of reception signals thus produced are output to the transmission and reception unit 14. In the present embodiment, a position and an orientation of the probe 12 are adjusted by the user so that the ultrasound is transmitted to and received from a head of the fetus which is the subject.

The transmission and reception unit 14 sends, to the probe 12, a plurality of transmission signals for exciting the plurality of transducers of the probe 12, to cause generation of the ultrasound at the probe 12. The transmission and reception unit 14 further executes a phasing addition on the plurality of reception signals produced by the plurality of transducers upon reception of the reflection echo, to form beam data arranged in a scanning direction of the ultrasound beam. The phasing addition is a process to electrically form a reception beam. Each beam data set is formed from a plurality of reflection echo signals arranged in a depth direction. As described, the transmission and reception unit 14 has a function of a transmission beam former and a function of a reception beam former. At the downstream of the transmission and reception unit, there is provided a beam data processor including a wave detection circuit or the like, which is not shown in the figures.

An ultrasound image former 16 is formed from, for example, a digital scan converter (DSC), and forms an ultrasound image as a living body image based on the plurality of beam data sets from the transmission and reception unit 14. In the present embodiment, the ultrasound image formed by the ultrasound image former 16 is a B-mode image, and is in particular an image showing a cross section of the head of the fetus. More specifically, the ultrasound image former 16 produces a display frame data array based on a reception frame data array. One reception frame data set is made of a plurality of beam data sets obtained by one beam scanning. The display frame data array forms the B-mode image as a video image. In the present embodiment, a sequence of ultrasound images sequentially produced by the ultrasound image former 16 are sent to a display processor 30 and an image storage 18.

The image storage 18 is a ring buffer which stores a plurality of ultrasound images formed by the ultrasound image former 16. For example, frames from the most recent frame to a predetermined time in the past are temporarily stored in the ring buffer. Alternatively, an image storage may be provided upstream of the ultrasound image former 16. In this case, a group of beam data sets before the processing performed by the ultrasound image former 16 is applied are stored for each frame. In the present embodiment, the measurement is not executed in real time during operation, but is executed during an image reproduction operation after freeze. More specifically, a plurality of ultrasound images stored in the image storage 18 are reproduced, and a user selects a particular ultrasound image from the reproduced images. The measurement of the fetal biparietal diameter is executed in a state where the ultrasound image is displayed on the screen as a static image.

A controller 20 is formed from, for example, a CPU or a microprocessor, and executes control of each part of the ultrasound diagnostic apparatus 10. The controller 20 has a plurality of functions which operate in the distance measurement, and these functions are shown in FIG. 1 as a plurality of blocks. These functions are realized in the present embodiment as software functions. Alternatively, these functions may be realized using hardware such as, for example, electric/electronic circuits, processors, or the like. Alternatively, these functions may be realized as a cooperation of hardware such as a CPU and a processor, and software (program). The blocks will now be described.

A standard range specifier 22 specifies a lower limit value and an upper limit value of a standard range of the fetal biparietal diameter which is the target of measurement. In the present embodiment, the standard range specifier 22 sets a normal range of the fetal biparietal diameter calculated from a statistical viewpoint as the standard range. Because the normal range of the fetal biparietal diameter differs depending on the number of weeks of pregnancy, the standard range specifier 22 specifies, as the standard range, a lower limit value and an upper limit value of the normal range of the fetal biparietal diameter according to the number of weeks of pregnancy. Specifically, the lower limit value and the upper limit value of the standard range of the fetal biparietal diameter are specified based on correspondence information showing a correspondence relationship between the lower limit value and the upper limit value of the normal range of the fetal biparietal diameter and the number of weeks of pregnancy, and the number of weeks of pregnancy which is input by the user. Alternatively, the lower limit value and the upper limit value of the standard range may be directly input by the user.

A graphic image former 24 forms various graphic elements displayed in an overlapped manner over the ultrasound image in the distance measurement on the ultrasound image. The graphic elements formed by the graphic image former 24 include a first movable marker which moves on the ultrasound image for designating a starting point (first measurement point) of the distance measurement, a second movable marker which moves on the ultrasound image for designating an end point (second measurement point) of the distance measurement, a standard range displaying figure which shows a standard range of the fetal biparietal diameter specified by the standard range specifier 22, or the like. The graphic elements are not limited to those described above, and an image for supporting the distance measurement, such as a line connecting the starting point marker and the second movable marker, may be formed. The group of formed graphic elements are overlapped over the ultrasound image by the display processor 30 and displayed on a display 32.

The standard range displaying figure showing the standard range of the fetal biparietal diameter is displayed on the display 32 before the starting point of the distance measurement is designated; that is, during the starting point designation. By displaying the standard range displaying figure before the starting point designation, it becomes possible to use the standard range displaying figure as an estimate for designating the starting point. In the present embodiment, a circular or an arc shaped (hereinafter, referred to as "circle-like shape") figure having the center at the first movable marker is used as the standard range displaying figure. With the use of the circle-like shape for the standard range displaying figure, there is shown a standard range of the fetal biparietal diameter with respect to a plurality of directions (if the shape is a circle, all directions) from the first movable maker.

In the present embodiment, the standard range displaying figure is formed from a double circular figure which includes a lower limit value circle centered at the first movable marker and having a radius of a value based on the lower limit value of the standard range specified by the standard range specifier 22, and an upper limit value circle centered at the first movable marker and having a radius of a value based on the upper limit value of the standard range specified by the standard range specifier 22. Specifically, the radii of the lower limit value circle and the upper limit value circle are determined in consideration of the lower limit value and the upper limit value of the specified standard range, and a display scale of the display 32.

As the standard range displaying figure, various structures may be employed, so long as the standard range of the fetal biparietal diameter is shown. For example, the lower limit value circle and the upper limit value circle described above may be shown with broken lines, or a portion between the lower limit value circle and the upper limit value circle may be colored in order to allow the user to more easily understand the standard range. Alternatively, in addition to the lower limit value circle and the upper limit value circle, an average value circle may be displayed, which is centered at the first movable marker and which has a radius of a value based on an average value of the fetal biparietal diameter.

In addition, when the starting point is designated, the display position of the standard range displaying figure is fixed at a position centered at the starting point position, and the standard range displaying figure continues to be displayed. With such a configuration, the user can use the circle-like shaped standard range displaying figure as an estimate in the positioning of the end point using the second movable marker.

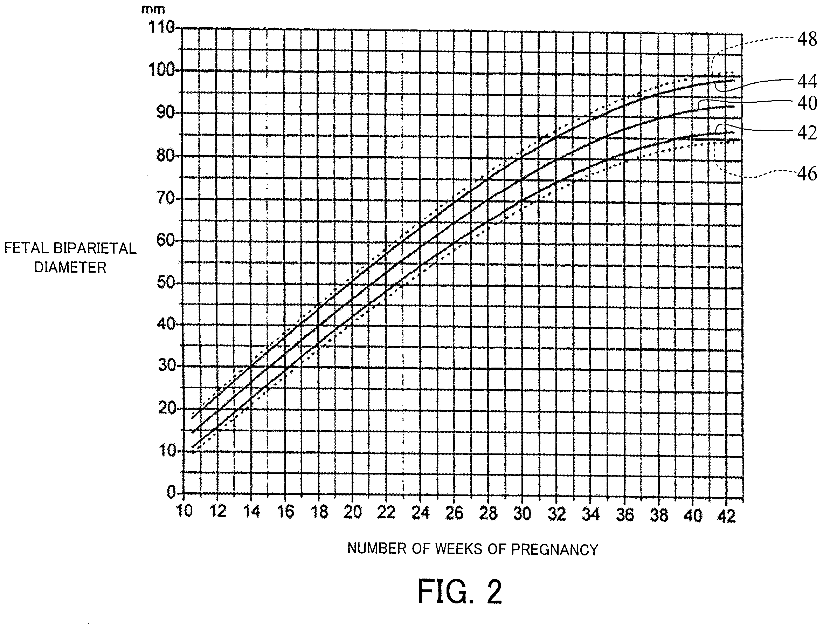

Details of the standard range displaying figure formed by the graphic image former 24 will be described later with reference to FIGs. 2 ∼ 7.

A measurement unit 26 calculates a distance between two measurement points designated on the ultrasound image. The two measurement points are designated by the user. Specifically, on the ultrasound image displayed on the display 32, the user moves the first movable marker using a trackball included in an inputter 34, and presses a determination button, to determine the starting point position of the distance measurement. Similarly, the second movable marker is moved and the determination button is pressed, to thereby determine the end point position. The measurement unit 26 measures the distance between two measurement points thus designated. The distance between the two measurement points is calculated in consideration of the distance on the ultrasound image and the display scale of the ultrasound image.

A storage 28 is, for example, a hard disk drive, a ROM, a RAM, or the like, and stores a program for operating various parts of the ultrasound diagnostic apparatus 10, calculation process results at the ultrasound diagnostic apparatus 10, or the like. The storage 28 also stores the correspondence information showing the correspondence relationship between the lower limit value and the upper limit value of the normal range of the fetal biparietal diameter and the number of weeks of pregnancy, which is referred to by the standard range specifier 22. The correspondence information may be stored as a function or in a form of a table or the like.

The display processor 30 executes a process to display, on the display 32, the ultrasound image formed by the ultrasound image former 16. In addition, as described above, the display processor 30 overlaps the group of graphic elements formed by the graphic image former 24 over the ultrasound image and displays the resulting image on the display 32. Further, the display processor 30 may display, on the display 32, a measurement result of the measurement unit 26 in a form or a report or the like.

The display 32 is, for example, a liquid crystal display, and displays the ultrasound image, the group of graphic elements, or the like. The inputter 34 includes a button, a switch, the trackball, or the like, and is used by the user for inputting the number of weeks of pregnancy of the pregnant woman, for moving the first and second movable markers, for designating the starting point and the end point of the distance measurement, or the like.

In the following, the details of the standard range displaying figure showing the standard range of the fetal biparietal diameter formed by the graphic image former 24 will be described with reference to FIGs. 2 ∼ 7 in connection to FIG. 1.

FIG. 2 is a diagram showing a function showing the relationship between the normal range of the fetal biparietal diameter and the number of weeks of pregnancy, stored in the storage 28. Of five graphs shown in FIG. 2, a graph 40 at the center is a graph showing an average value of the fetal biparietal diameter in each week of pregnancy. A graph 42 below the graph 40 is a graph showing (the average - 1.5SD (Standard Deviation)), and a graph 44 above the graph 40 is a graph showing (the average + 1.5SD). These graphs are calculated based on a measurement results of the fetal biparietal diameter in the past. In each week of pregnancy, about 86% of the entirety of fetuses are included between -1.5SD and +1.5SD. For example, in the 18th week of pregnancy, about 86.6% of the entirety of fetuses have a size of fetal biparietal diameter in a range from about 35 mm to about 44 mm. Thus, in the present embodiment, the range of ±1.5SD is set as the standard range of the fetal biparietal diameter, with the value at -1.5SD as the lower limit value of the standard range and the value at +1.5SD as the upper limit value of the standard range. The standard range specifier 22 specifies the upper limit value and the lower limit value of the standard range from the function shown in FIG. 2, based on the number of weeks of pregnancy which is input by the user.

A graph 46 positioned further below the graph 42 showing -1.5SD is a graph showing (the average - 2.0SD), and a graph 48 positioned further above the graph 44 showing +1.5SD is a graph showing (the average + 2.0SD). In the present embodiment, the range of ±1.5SD is set as the standard range for the measurement of the fetal biparietal diameter, but the standard range is not limited to the range of ±1.5SD. Currently, in Japan, a criterion for the standard range in the measurement of the fetal biparietal diameter is ±1.5SD, but the criterion for the standard range differs from country to country, and may change even in Japan in the future. In addition, in measurements other than the measurement of fetal biparietal diameter, ranges other than +1.5SD may be set as the standard range. The standard range may be set according to these criteria.

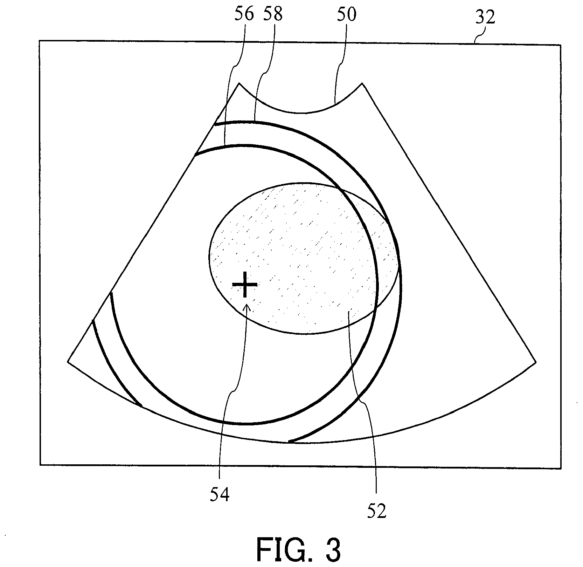

FIG. 3 is a diagram showing an example display of the standard range displaying figure showing the standard range of the fetal biparietal diameter. As shown in FIG. 3, on the display 32, a B-mode image 50 formed by the ultrasound image former 16 is displayed. The B-mode image 50 includes a cross section 52 of the head of the fetus. The head cross section 52 is a cross section viewed from the side of the top of the head, with a front surface of the face shown at the left. Therefore, the left side temporal region of the fetus is at a lower side of the head cross section 42, and the right side temporal region is at an upper side of the head cross section 52. In the measurement of the fetal biparietal diameter, the distance between the left and right side temporal regions is measured. Thus, in the example display of FIG. 3, a candidate position for the starting point is near a center at the lower side of the outline of the head cross section 52 (or near a center at the upper side), and a candidate position of the end point is near the center at the upper side of the outline of the head cross section 52 (or near the center at the lower side when the starting point is near the center at the upper side).

When the distance measurement is started, a first movable marker 54 for designating the starting point of the distance measurement is displayed on the display 32. At the same time, there are displayed a lower limit value circle 56 and an upper limit value circle 58 having a circle-like shape centered at the first movable marker 54. As described above, the radius of the lower limit value circle 56 is a value based on the lower limit value of the standard range of the fetal biparietal diameter, and the radius of the upper limit value circle 58 is a value based on the upper limit value of the standard range. In other words, a position in which a distance from the first movable marker 54 shows the lower limit value of the standard range of the fetal biparietal diameter and a position in which the distance shows the upper limit value are displayed for a plurality of directions.

As shown in FIG. 3, in the present embodiment, of the lower limit value circle 56 and the upper limit value circle 58, portions extending beyond the display range of the B-mode image 50 are not displayed. With such a configuration, it is possible to prevent blockage, by the lower limit value circle 56 and the upper limit value circle 58, of the display of various types of information displayed outside of the display range of the B-mode image 50.

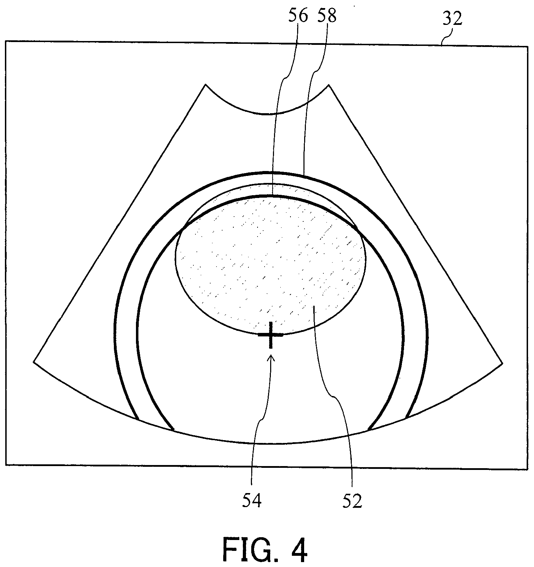

FIG. 4 shows an example display when the first movable marker 54 is moved to the starting point candidate position. The lower limit value circle 56 and the upper limit value circle 58 move in a manner to follow the movement of the first movable marker 54, and relative positional relationship among these elements is maintained. Therefore, when the first movable marker 54 is moved to the starting point candidate position, lines having the distance from the starting point candidate position as the lower limit value and the upper limit value of the standard range are shown by the lower limit value circle 56 and the upper limit value circle 58.

In this state, a position of the center of the right side temporal region (near the center of the upper side of the outline of the head cross section 52) which is the end point candidate position and positions of the lower limit value circle 56 and the upper limit value circle 58 are compared, so as to allow judgment of whether or not the current position of the first movable marker 54 is appropriate as a starting point of the distance measurement.

When the end point candidate position is located between the lower limit value circle 56 and the upper limit value circle 58, it is possible to comprehend that the fetal biparietal diameter of the fetus which is the subject falls within the standard range before the starting point position is designated. That is, this means that the current position of the first movable marker 54 is at an appropriate position as the starting point of the distance measurement.

In contrast, if the end point candidate position is not located between the lower limit value circle 56 and the upper limit value circle 58, various possibilities may be considered. First, a case may be considered in which the current position of the first movable marker 54 is not at an appropriate position as the starting point. Secondly, a case may be considered in which the head cross section 52 included in the B-mode image 50 is not an appropriate cross section. Thirdly, a case may be considered in which the size of the fetus actually falls outside the standard range. When the size of the fetus is not normal, it is typical to again capture the ultrasound image and again measure the size. In other words, in any of the above-described cases, superior measurement result cannot be obtained when the current position of the first movable marker 54 is set as the starting point for the distance measurement. Therefore, when the end point candidate position is not located between the lower limit value circle 56 and the upper limit value circle 58, the user can comprehend that the current position of the first movable marker 54 is not at an appropriate position as the starting point of the distance measurement.

In this manner, according to the present embodiment, the user can comprehend whether or not the starting point candidate position is at an appropriate position as the starting point of the distance measurement, before the starting point of the distance measurement is designated. When the starting point candidate position is not appropriate as the starting point of the distance measurement, the user can comprehend it at an early stage, and can execute countermeasures such as review of the starting point candidate position and re-selection or re-capturing of the ultrasound image at an early stage. Thus, it is possible to reduce the amount of re-doing work by the user, and to reduce the work effort related to the distance measurement.

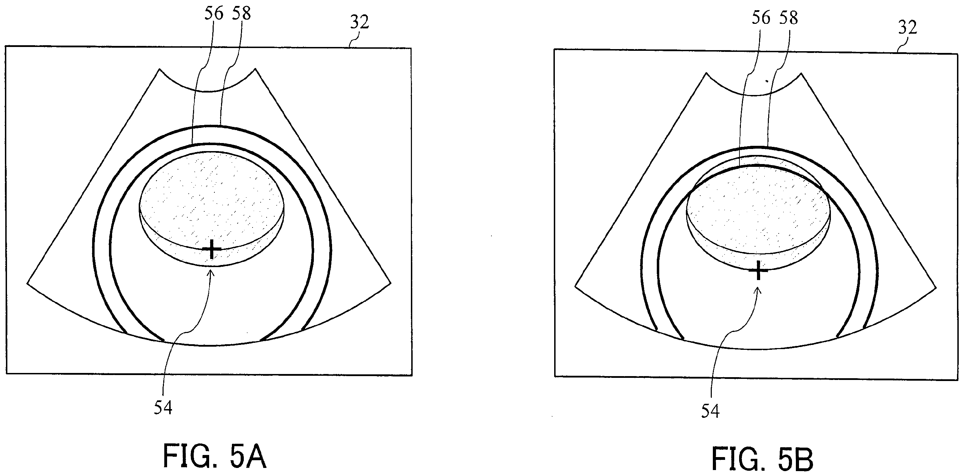

The lower limit value circle 56 and the upper limit value circle 58 can be used, in addition to the judgment of whether or not the designated starting point candidate position is at an appropriate position, as an estimate when the position of the starting point candidate position is to be determined. FIG. 5 is a diagram showing an example of another usage of the lower limit value circle 56 and the upper limit value circle 58. For example, as shown in FIGs. 5A and 5B, there may be cases where the outline at the center of the left side temporal region is unclear due to influences of an artifact or the like, and the outline appears as a double line. In this case, it is possible to deduce which of the outlines is the correct outline position by moving the first movable marker 54 over the two outlines. That is, in a case where, when the first movable marker 54 is moved to the first outline of the two outlines as shown in FIG. 5A, the center of the right side temporal region is not located between the lower limit value circle 56 and the upper limit value circle 58, but when the first movable marker 54 is moved over the second outline as shown in FIG. 5B, the center of the right side temporal region is located between the lower limit value circle 56 and the upper limit value circle 58, it can be deduced that the second outline is the correct outline of the center of the left side temporal region.

In the present embodiment, as a two-dimensional figure showing the standard range of the fetal biparietal diameter, the lower limit value circle 56 and the upper limit value circle 58 are displayed. This is because it is desirable to show the standard range of the fetal biparietal diameter in all directions from the first movable marker 54, because the controller 20 cannot comprehend, before the starting point is designated, which direction from the first movable marker 54 the distance measurement is to be executed. Alternatively, the display portion of the lower limit value circle 56 and the upper limit value circle 58 may be limited according to the position of the first movable marker 54 or the like.

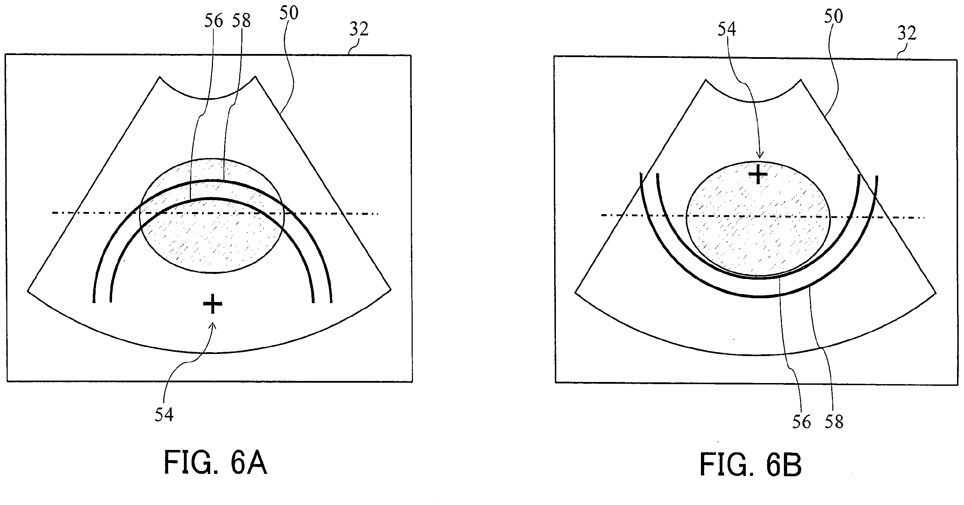

FIG. 6 is a diagram showing an example of another display form of the lower limit value circle 56 and the upper limit value circle 58. For example, when the first movable marker 54 is positioned at a lower side in relation to a center line of a display region of the B-mode image 50, it is more likely that the direction of the distance measurement is a direction toward the upper side than the first movable marker 54. Therefore, in this case, it is sufficient to show the standard range of the fetal biparietal diameter only in the region above the first movable marker 54. Thus, as shown in FIG. 6A, a configuration may be employed in which only the upper halves of the lower limit value circle 56 and the lower limit value circle 58 are displayed when the first movable marker is at a position lower than the center line of the display region of the B-mode image 50. Similarly, as shown in FIG. 6B, a configuration may be employed in which only the lower halves of the lower limit value circle 56 and the upper limit value circle 58 are displayed when the first movable marker is at a position above the center line of the display region of the B-mode image 50. In addition, only the right halves of the lower limit value circle 56 and the upper limit value circle 58 may be displayed when the first movable marker is at a left side of the center line of the display region of the B-mode image 50, and only the left halves of the lower limit value circle 56 and the upper limit value circle 58 may be displayed when the first movable marker is at a position right of the center line of the display region of the B-mode image 50. Alternatively, the display portions of the lower limit value circle 56 and the upper limit value circle 58 may be designated by the user.

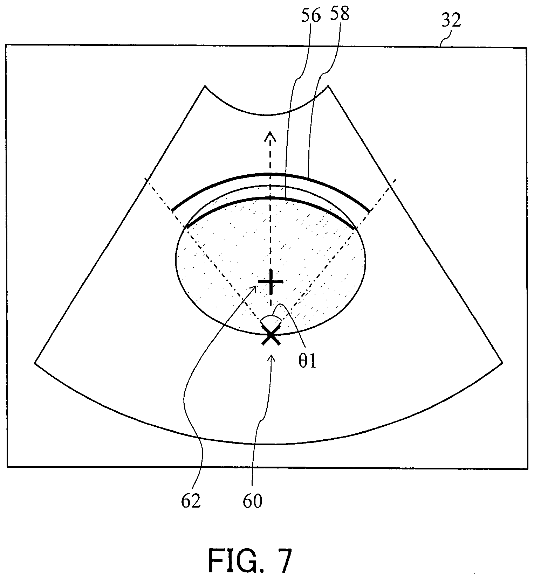

FIG. 7 is a diagram showing an example display after the starting point is designated. When the starting point is designated by the user pressing the determination button or the like, the first movable marker is fixed at the designated starting point position. In the present embodiment, in order to distinguish between the first movable marker which is being moved (that is, before the starting point is designated) and the first movable marker after fixation (hereinafter referred to as "starting point marker"), the shapes of the first movable markers are set different from each other. When the starting point is designated, the display positions of the lower limit value circle 56 and the upper limit value circle 58 are fixed at positions centered at the starting point marker 60.

When the user operates the trackball after the starting point is designated, a second movable marker 62 for designating the end point of the distance measurement is displayed. In the present embodiment, the shape of the second movable marker 62 is set identical to the first movable marker 54, but alternatively, the shapes may be different from each other. Even after the second movable marker 62 is displayed; that is, even at the stage of designating the end point, the lower limit value circle 56 and the upper limit value circle 58 continue to be displayed. Thus, the user can use the lower limit value circle 56 and the upper limit value circle 58 as an estimate for the positioning of the end point.

When the second movable marker 62 is moved, the controller 20 can comprehend the direction of the distance measurement by the position relationship between the starting point marker 60 and the second marker 62. That is, the controller 20 comprehends that a direction extending from the starting point marker 60 to the second movable marker 62 as the direction of distance measurement. Because it is sufficient that the lower limit value circle 56 and the upper limit value circle 58 are displayed only in the direction of the distance measurement, the display processor 30 limits the display ranges of the lower limit value circle 56 and the upper limit value circle 58 according to the direction of the distance measurement.

More specifically, as shown in FIG. 7, the display processor 30 limits the display of the lower limit value circle 56 and the upper limit value circle 58 to a range of a certain angel θ1 centered around the direction of the distance measurement (an arrow of a broken line in the figure) deduced from the position relationship between the starting point marker 60 and the second movable marker 62. With such a configuration, a portion to be used as the estimate for positioning the end point can be left while deleting the display of the other, unnecessary portions, resulting in resolving of complexity of display

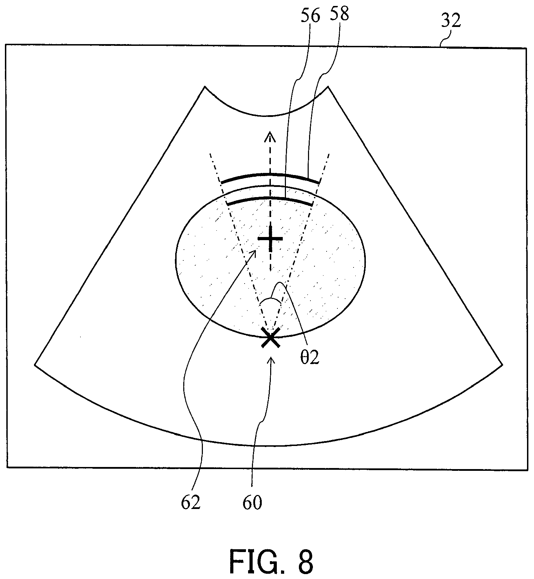

FIG. 8 is a diagram showing an example display when the second movable marker is further moved. The display processor 30 changes display angle ranges of the lower limit value circle 56 and the upper limit value circle 58 according to the distance between the starting point marker 60 and the second movable marker 62. Specifically, the display angle is reduced as the distance between the starting point marker 60 and the second movable marker 62 is increased. In the example display of FIG. 8, the distance between the starting point marker 60 and the second movable marker 62 is greater than in FIG. 7. Therefore, the display angle θ2 in FIG. 8 is smaller than θ1.

In order that the lower limit value circle 56 and the upper limit value circle 58 function as the estimate for the positioning of the end point, a lower limit value is set for the display angle ranges of the lower limit value circle 56 and the upper limit value circle 58. Even when the distance between the starting point marker 60 and the second movable marker 62 becomes larger, the display angle ranges of the lower limit value circle 56 and the upper limit value circle 58 are configured to not become lower than the lower limit value.

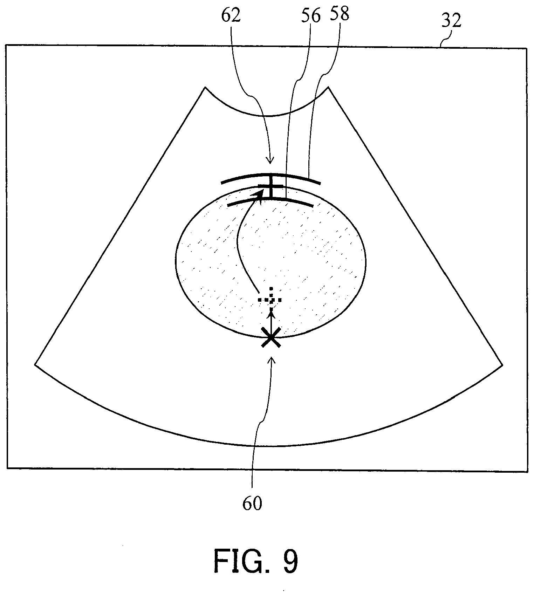

FIG. 9 is a diagram showing an alternative example movement method of the second movable marker 62. As described above, when the second movable marker 62 is moved, the controller 20 can comprehend the direction of the distance measurement by the positional relationship between the starting point marker 60 and the second movable marker 62. Further, the controller 20 can comprehend that an intermediate point between the lower limit value circle 56 and the upper limit value circle 58 in the comprehended direction of the distance measurement is a strong candidate position of the end point of the distance measurement. Therefore, the display processor 30 causes, according to an instruction from the controller 20 and when the second movable marker 62 starts to move from the starting point marker 60, the second movable marker 62 to jump to a range between the lower limit value circle 56 and the upper limit value circle 58 in the movement direction of the second movable marker 62. Desirably, the second movable marker 62 is caused to jump at or near an intermediate point of the lower limit value circle 56 and the upper limit value circle 58. The timing of jumping the second movable marker 62 may be determined according to the distance between the starting point marker 60 and the second movable marker 62. For example, the second movable marker 62 is caused to jump when the second movable marker 62 is moved from the starting point marker 60 in one direction for a predetermined distance. Alternatively, the second movable marker 62 may be caused to jump when the second movable marker 62 is moved from the starting point marker 60 in one direction for a predetermined period of time. By causing the second movable marker 62 to jump, it is possible to further reduce the work effort of the user for designating the end point of the distance measurement.

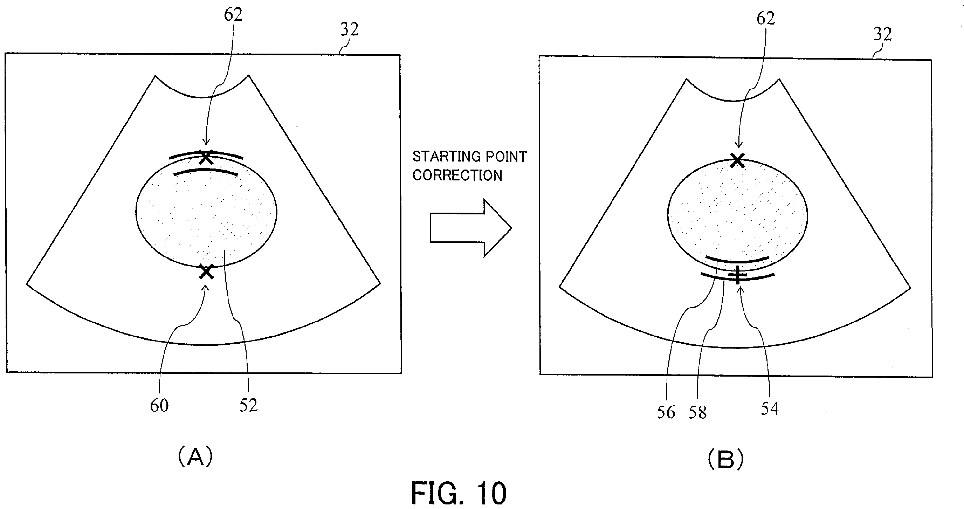

FIG. 10 is a diagram showing an example display when the starting point is corrected after the end point is designated. The ultrasound diagnostic apparatus 10 has a function to correct the designated starting point or end point. In the following, there will be described an example display when the starting point is corrected after the end point is designated. When the designated starting point position is to be corrected before the end point is designated, referring again to the display of FIG. 3, the user again designates the starting point with the first movable marker 54. When the designated end point position is to be corrected, referring again to the display of FIG. 7, the user again designates the end point with the second movable marker 62.

As shown in (A) of FIG. 10, when the end point is designated, an end point marker 64 is displayed at the designated end point position. Then, when the user notices that the position of the starting point marker 60 is deviated from an outline position of the head cross section 52 and instructs correction of the starting point position by pressing a correction button included in the inputter 34, as shown in (B) of FIG. 10, the display processor 30 deletes the starting point marker 60, and again displays the first movable marker 54 at or near the position where the starting point marker 60 was displayed. Further, the display processor 30 again displays the lower limit value circle 5+6 and the upper limit value circle 58. In this case, the displayed lower limit value circle 56 and upper limit value circle 58 are displayed at positions centered at the end point marker 64. With such a configuration, the user can correct the starting point position using the lower limit value circle 56 and the upper limit value circle 58 as an estimate. Similar to the examples shown in FIG. 7 or 8, the display angle ranges of the lower limit value circle 56 and the upper limit value circle 58 change according to the distance between the end point marker 64 and the first movable marker 54.

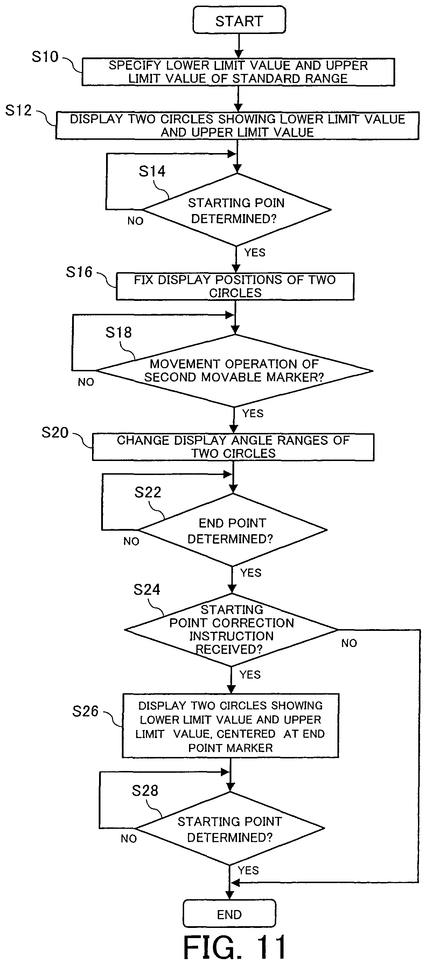

FIG. 11 is a flowchart showing another example flow of operations of the ultrasound diagnostic apparatus 10. The steps shown in FIG. 11 will now be described with reference to FIG. 1.

When the user designates the ultrasound image to be used for distance measurement and the distance measurement is started in the ultrasound diagnostic apparatus 10, in step S10, the standard range specifier 22 specifies the lower limit value and the upper limit value of the standard range of the fetal biparietal diameter of the fetus based on the number of weeks of pregnancy of the pregnant woman which is input by the user.

In step S12, the display processor 30 displays, on the display 32, the first movable marker for designating the starting point of the distance measurement. Further, the display processor displays two circle-like shaped figures centered at the first movable marker and having radii of values based on the lower limit value and the upper limit value specified in step S10. The formed figures are overlapped over the ultrasound image and displayed.

In step S 14, the controller 20 judges whether or not the user has designated the starting point. Until the starting point is designated, the display processor 30 continues to display the first movable marker and the two circle-like shaped figures.

When the starting point is designated, in step S 16, the display processor 30 displays the starting point marker at the starting point designated position, and further fixes the display positions of the two circle-like shaped figures at positions centered at the starting point marker.

In step S18, the controller 20 judges whether or not the user has operated the trackball. That is, the controller 20 judges whether or not the movement operation of the second movable marker for designating the end point position is executed.

When the user operates the trackball, in step S20, the display processor 30 displays the second movable marker. Further, the display processor 30 limits the display ranges of the two circle-like shaped figures in a certain angle range centered around the direction extending from the starting point marker to the second movable marker.

In step S22, the controller 20 judges whether or not the user has designated the end point. Until the end point is designated, the display processor 30 continues to display the second movable marker and the two circle-like shaped figures centered at the starting point marker while changing the display ranges thereof. When the end point position is designated, the display processor 30 deletes the second movable marker, and displays the end point marker at the designated end point position.

In step S24, the controller 20 judges whether or not the controller 20 has received from the user a correction instruction of the designated measurement points. In the present flowchart, a case where the correction instruction of the starting point is received from the user will be described in particular. When the correction instruction by the user is not received, the measurement unit 26 measures the distance between the designated starting point and the designated end point.

When the correction instruction of the starting point by the user is received, in step S26, the display processor 30 deletes the starting point marker and again displays the first movable marker. Further, the display processor 30 displays two circle-like shaped figures centered at the end point marker and having the distance from the end point marker as the lower limit value and the upper limit value of the standard range of the fetal biparietal diameter.

In step S28, the controller 20 judges whether or not the starting point is again designated by the user. Until the starting point is designated, the display processor 30 continues to display the first movable marker and the two circle-like shaped figures centered at the end point marker. When the starting point is again designated in step S28, the measurement unit 26 measures the distance between the designated starting point and the designated end point.

In the present embodiment, measurement of the fetal biparietal diameter is exemplified, but the present disclosure is not limited to the measurement of the fetal biparietal diameter, and may be applied to any measurement of a distance between two points designated on an ultrasound image. In this case, as the table or function showing the lower limit value and the upper limit value of the standard range stored in the storage 28, a table or a function corresponding to the measurement target is prepared.

In addition, in the present embodiment, the ultrasound diagnostic apparatus 10 is exemplified as the ultrasound image processor. Alternatively, as the ultrasound image processor, for example, a PC or the like may be used. In this case, the ultrasound image formed by the ultrasound diagnostic apparatus is sent to the PC, and the PC executes the specification of the standard range, the overlapping of the graphic image, the distance measurement, or the like.

### REFERENCE SIGNS LIST

10 ULTRASOUND DIAGNOSTIC APPARATUS; 12 PROBE; 14 TRANSMISSION AND RECEPTION UNIT; 16 ULTRASOUND IMAGE FORMER; 18 IMAGE STORAGE; 20 CONTROLLER; 22 STANDARD RANGE SPECIFIER; 24 GRAPHIC IMAGE FORMER; 26 MEASUREMENT UNIT; 28 STORAGE; 30 DISPLAY PROCESSOR; 32 DISPLAY; 34 INPUTTER.

## Claims

1. An ultrasound image processor comprising:
a measurement unit that measures a distance between a first measurement point and a second measurement point which are sequentially designated on an ultrasound image;
a first marker displaying unit that displays a first marker which moves on the ultrasound image in response to a user operation for designating the first measurement point; and
an index figure displaying unit that displays, at least before the first measurement point is designated and on the ultrasound image, an index figure showing a standard range of a distance between the first measurement point and the second measurement point with the first marker as a point of reference.

2. The ultrasound image processor according to Claim 1, wherein
the index figure includes a first two-dimensional shape having a circular shape or an arc shape centered at the first marker and having a lower limit value of the standard range as a radius, and a second two-dimensional shape having a circular shape or an arc shape centered at the first marker and having an upper limit value of the standard range as a radius.

3. The ultrasound image processor according to claim 1 or 2, wherein
the index figure displaying unit moves the index figure in a manner to follow a movement of the first marker.

4. The ultrasound image processor according to Claim 2, further comprising:
a second marker displaying unit that displays a second marker which moves on the ultrasound image in response to a user operation for designating the second measurement point after the first measurement point is designated, wherein
the index figure displaying unit fixes display positions of the first two-dimensional shape and the second two-dimensional shape when the first measurement point is designated, and limits, when the second marker is moved and after the display positions are fixed, display ranges of the first two-dimensional shape and the second two-dimensional shape to an angle range centered around a direction from the first measurement point and through a display position of the second marker.

5. The ultrasound image processor according to Claim 4, wherein
the index figure displaying unit reduces the angle range as a distance between the first measurement point and the second marker is increased.

6. The ultrasound image processor according to Claim 4, wherein
the second marker displaying unit initially displays the second marker at the designated first measurement point, and moves, when the second marker is moved, the second marker in a direction of movement of the second marker from the first measurement point and to a range between the first two-dimensional shape and the second two-dimensional shape.
